# EUROPEAN PATENT APPLICATION

(11) **EP 1 477 172 A1**
(43) Date of publication of application: **17.11.2004**
(21) Application number: 04252711.9
(22) Date of filing: 11.05.2004
(51) Int. Cl.: A61K 31/7004, A61P 31/04

(54) **Treatment of shipping fever**

(30) Priority: 16.05.2003 US 439771
(71) Applicant: Jones, Alonzo, Plainview, Texas 79072 (US); Bozeman, Jerry, Plainview, Texas 79072 (US)
(72) Inventor: Jones, Alonzo, Plainview, Texas 79072 (US); Bozeman, Jerry, Plainview, Texas 79072 (US)
(74) Representative: Banford, Paul Clifford

(57) **Abstract**

A present invention concerns the use of xylitol for the reduction or prevention of the occurrence of respiratory infections, and other ailments associated with close quartering of non-human mammals such as cattle, the ailments generally referred to as "shipping fever."

## Description

The present invention is directed generally to the use of xylitol for treating non-human mammals, such as cattle. More particularly, embodiments of the invention are directed to treating illness in cattle associated with transportation and other close-quarter contact.

Large mammals, like humans, are subject to becoming ill because of exposure to bacteria and viruses. For instance, Cattle on pasture land may experience fewer illnesses because, in part, of the limited population of other animals to which they are exposed. However, if these cattle come in contact with animals carrying bacteria and viruses to which they have not previously been exposed, illness based on the new strains is likely.

At times in the life cycle of cattle, they may be moved from place to place, and in the process brought into contact with and exposed to different animals. More particularly, at some point cattle may be moved to a sale barn, where prior to the sale they are held in small pens and exposed to unfamiliar cattle. From the sale barn, cattle from disparate locations may be combined into a group or lot and moved to other locations, like a feed lot or other pasture lands. During these movements, the cattle may be tightly packed in truck trailers. In fact, cattle are often intentionally placed in the trucks in such a manner that the animals have very little room for movement, thus ensuring that no animals fall down where they may be subsequently trampled.

Cattle held in close-quarters, such as a group of cattle in a holding pen at a sale barn or within a trailer during shipping, tend to develop various illnesses thereafter. As an example, consider cattle shipped to a feed lot. On average, 25% percent of cattle (which may weigh in the range of 500 to 600 pound - approximately 230kg - 275 kg) entering a feed lot may experience some type of illness requiring treatment. Note that this is an average, and in some cases very little illness for a group of cattle may be experienced, yet in other cases an entire group of cattle may experience illness. Moreover, death loss, again on average, may be between 5% and 10%.

While there may be many reasons that cattle may become ill, such as contact with bacteria and virus to which they have not previously been exposed, the mere act of placing the cattle together for shipping (even a closed group of cattle) may cause many of the animals to develop illnesses such as fever associated with respiratory infections. The illnesses triggered by shipping of otherwise healthy cattle may be termed "shipping fever." Shipping fever may account for half or more of the illness experienced by cattle, and therefore may account for between 2.5% to 5% of the death loss of cattle entering a feed lot. At a price per animal of approximately US $500 at this weight range (in the USA in 2003), each lost animal may severely affect profitability.

It is therefore an object of the present invention to provide ways of reducing illness in non-human mammals, such as cattle, associated with shipping and other close-quarter contact.

According to a first aspect of the present invention there is provided the use of xylitol in the manufacture of a medicament for treating shipping fever and/or ailments associated with close quartering of a non-human mammal.

Embodiments of the invention may be directed to reducing the occurrence of respiratory infections, and other illnesses generally referred as "shipping fever," in non-human mammals such as cattle by administering xylitol. The xylitol may be delivered in a solution, and may be applied in mist form directly to one or more nostrils of the non-human animal. Alternatively, the solution comprising xylitol may be applied in mist form in the atmosphere surrounding the non-human mammal.

Xylitol may be used according to the invention to overcome the deficiencies of the prior art. The various characteristics described above, as well as other features, will be readily apparent to those skilled in the art upon reading the following detailed description.

Certain terms are used throughout the following description and claims to refer to particular system components, substances and features. This document does not intend to distinguish between components, substances and/or features that differ in name but not function.

In the following discussion and in the claims, the terms "including" and "comprising" are used in an open-ended fashion, and thus should be interpreted to mean "including, but not limited to...". In the context of the present invention a large animal veterinarian is considered to have ordinary skill in this art.

Xylitol may be used according to the first aspect of the invention to reduce shipping fever in non-human mammals, such as cattle. The invention was developed in the context of cattle and the following description is based on that developmental context. However, it will be appreciated that discussion of the various embodiments with respect to cattle should not be construed as limiting the uses disclosed herein to cattle only. Uses of xylitol, as described herein, may be equally applicable to many non-human animals (e.g. sheep, pigs, horses and the like). Furthermore, it should be understood however that the term "shipping fever" is not necessarily limited to illnesses associated with transportation of animals, but may also comprise illnesses associated with close quartering of animals, whether because of shipping constraints or space limitations in holding facilities.

The inventors of the present specification have discovered at least one cause of illness normally associated with the term "shipping fever," and thus have also a devised ways to reduce or prevent such illness. Although they do not wish to be bound by any hypothesis, they believe that the usefulness of xylitol may, at least in part, be explained by the position animals are forced to stand in when being shipped or penned in close quarters. When cattle are on pasture lands, or held in large areas, their normal stance is head prone. For example, as the cattle graze, or chew cud, their heads, including their nasal cavities, may be generally pointed downward. This head prone stance may allow nasal fluids, including bacteria in the nasal fluid, to exit by way of the nostrils.

In situations where the cattle are in close quarters, however, the head-prone stance may not be achievable. For example, in preparation for working the cattle or placing the cattle in trailers for shipping, the cattle may be held in a squeeze pen - a pen where very little movement of the cattle is possible. During shipping, cattle may be placed in the trailer in a close-packed manner which, as previously mentioned, may ensure that no animals fall, or lay down, and thus become subject to being trampled. However, holding the cattle together in this manner may also cause the cattle to hold their heads supine, allowing nasal fluid to run into the throat, and further allowing aspiration of the nasal fluid into the lungs. Nasal fluid may normally contain bacteria, but the problem may be exacerbated to some extent by feces and urine near the nose and mouth caused by the close quartering. Thus, ailments categorized as "shipping fever" may include fever associated with respiratory infections and pneumonia.

Embodiments of the present invention may reduce the occurrence of illness associated with shipping fever by treating the cattle with a solution comprising xylitol at one or more times including before shipping, during shipping, or after shipping. Alternative embodiments of the present invention may prevent or reduce the occurrence of shipping fever-like ailments associated with close quartering of cattle by treating the cattle with a solution comprising xylitol at one or more times including before, after and/or during the close quartering. Preferably, however, the xylitol may be administered after the trip.

Xylitol is the alcohol form of xylose, and is commonly used as a sweetener in food products. Xylitol also has the property that several pathogenic bacteria exposed to xylitol are less adherent to their environment. However xylitol is not known as an antibiotic. This makes the efficacy of xylitol according to the present invention a surprising development.

A co-inventor of the present specification holds a patent for the use of xylitol in solution for treating humans, U.S. Patent No. 6,054,143 titled "Xylitol Delivery," issued April 25, 2000, incorporated by reference herein as if reproduced in full below, as well as a patent for xylitol in solution, U.S. Patent No. 6,258,372 titled "Xylitol Nose Spray," issued July 10, 2001, also incorporated by reference herein as if reproduced in full below. It will be appreciated that the means for delivering xylitol and the xylitol solutions described in these patents may be used according to the present invention.

Embodiments of the present invention are directed to treating cattle with xylitol in solution in order to reduce or prevent shipping fever, and/or ailments associated with close quartering of cattle. The solution may be a saline based solution for some embodiments, but the solution may also have just a water base. A maximum strength may be a saturated solution comprising approximately of 64 grams of xylitol per 100mls of solution. The inventor has yet to determine a precise lower limit of xylitol that would be effective, but as little as 1% xylitol in solution has shown to be effective. It should be understood however, that a 1% xylitol in solution should not be construed as a lower limit of effectiveness; rather, this is only the lower limit of tested effectiveness. Smaller percentages of the xylitol in solution may likewise be effective, and thus their use would be within the scope and spirit of the invention.

The precise mode of application of the xylitol in solution may vary substantially. In at least some embodiments, nostrils of the cattle may be treated directly prior to close quartering, such as shipping. In particular, in these embodiments the xylitol in solution be applied to each nostril of each animal in the form of a mist (nebulized), which application preferably takes place for approximately two respiratory cycles of the animal for each nostril. In these embodiments, between approximately 0.005 ml to 500 mls of a solution comprising xylitol and a carrier (e.g saline or water) may be administered to the nostril of the non-human mammal; preferably about two to five US fluid ounces (approximately 50 - 150mls)is administered; and more preferably four to five US fluid ounces (approximately 100 - 150mls) may be applied to each animal. The dose may be approximately equally divided between the two nostrils.

In yet other embodiments, the cattle may be treated by misting in each nostril for approximately two respiratory cycles after the close quartering has ended, e.g. after unloading the animals at their destination, such as a feed yard. Note that embodiments that pre-treat the animals, and the embodiments that post-treat the animals, are not mutually exclusive - the animals may be treated in this manner both before and after the close quartering situation. In these direct treatment embodiments, because of relatively low volumes of solution used as compared to other embodiments (discussed below), the solution may be a saline solution.

In yet other embodiments, the xylitol in solution may be applied to the animals during close quartering. For example, the xylitol may be administered during shipping, possibly within the shipping chamber such as a trailer. In the location of the close quartering, whether a cattle trailer for transportation or squeeze pen, the xylitol in solution is preferably misted or nebulized into the atmosphere near (preferably just above) the cattle, thus forcing the animal to breath the solution. In the embodiments where the animals are treated on a mass scale, a total amount of xylitol in solution nebulized preferably amounts to one-half pound of solution (approximately 225mls) for each animal. For example, and without limitation, a set of fifty cows in a cattle trailer would preferably be misted with approximately 3 gallons (approximately 11 litres) of solution. Application of the solution in a mist may take place by a hand held device, similar to a yard chemical sprayer-type device. Alternatively, a mist system may be permanently or semi-permanently mounted in the area of the close quartering, e.g. within the cattle trailer and may be similar in form to water misting systems for cooling purposes. In the non-limiting example of a mist system in a cattle trailer, the cattle may be treated with xylitol in solution at any point in the journey, or at multiple points. Preferably, however, the xylitol in solution is applied at or near the destination.

When used to treat cattle, it will be appreciated that the xylitol may be administered to each of the cattle on an individual basis before loading the cattle for shipping. Alternatively the xylitol may be given to each of the cattle on an individual basis after unloading from shipping. It will also be appreciated that a mist, comprising xylitol, may be administered to the cattle as a group within a shipping chamber.

A preferred solution of xylitol for use in mist form may comprise between approximately one and 64 parts xylitol, and 100 parts water.

It will be appreciated that xylitol is useful for treating shipping fever and associated illnesses (i.e. when symptoms are manifest) or as a prophylatic to prevent the development of shipping fever and associated diseases.

By way of example, the inventors formulated a solution of xylitol (96 grammes in 150mls of sterile water) for administration directly to a nostril of a mammal as a mist. This formulation was used to assess whether or not shipping fever and associated illnesses may be reduced in cattle penned in close quarters. The solution was applied directly to the nostrils of the cattle for approximately two respiratory cycles of the animal. This was done before and/or after penning the cattle in close quarters. Treated cattle were found to suffer less illness than untreated animals.

By way of further example a solution of xylitol (96 grammes in 150mls of sterile water) was delivered, by nebulizing the solution, as a mist in the atmosphere proximate to penned cattle. 3 Gallons (approximately 11 litres) was delivered to 50 penned cattle. Treated cattle were found to suffer less illness than untreated animals.

The above discussion is meant to be illustrative of the principles and various embodiments of the present invention. Numerous variations and modifications will become apparent to those skilled in the art once the above disclosure is fully appreciated. For example, the precise time of application of the xylitol in solution may vary appreciably, and yet the benefits still obtained. Further, the various application methods may be used singularly, or in various combinations, to achieve the desired effect. Further still, while the specification has focused on the treatment of cattle, other mammals such as buffalo, horses, sheep, goats, dogs, cats and the like, may benefit treatments of xylitol in solution as well. Application volumes for this spectrum of animals may range from 0.005 mls for small animals to 500 mls for large animals. Finally, while the various embodiments may use mists or nebulized solution, the xylitol in solution may likewise be applied in a purely aqueous form, and yet the benefits still obtained.

## Claims

1. The use of xylitol in the manufacture of a medicament for treating shipping fever and/or ailments associated with close quartering of a non-human mammal.

2. The use according to claim 1 wherein the xylitol is formulated for administration as a mist that enters through a nostril of the non-human mammal.

3. The use according to claim 2 wherein the xylitol is formulated for administration as a mist directly into a nostril of the non-human mammal.

4. The use according to claim 3 wherein the medicament is a solution of approximately 0.005mls to 500mls comprising xylitol and a pharmaceutically acceptable carrier.

5. The use according to claim 4 wherein the medicament is a solution of approximately 100 - 150mls comprising xylitol and a pharmaceutically acceptable carrier.

6. The use according to any one of claims 3 -5 wherein the xylitol is for administration to the nostril for approximately two respiratory cycles.

7. The use according to any preceding claim wherein the xylitol is for administration to individual mammals before loading the mammals for shipping.

8. The use according to any one of claims 1 - 6 wherein the xylitol is for administration to individual mammals after unloading from shipping.

9. The use according to claim 2 further wherein the xylitol is formulated for administration as a mist in the atmosphere proximate to the non-human mammal.

10. The use according to claim 9 wherein the medicament comprises approximately 225mls of xylitol and a pharmaceutically acceptable carrier for misting into the atmosphere surrounding each non-human mammal.

11. The use according to claims 8 or 9 wherein the xylitol is for administration to the mammals as a group within a shipping chamber or pen.

12. The use according to any one of claims 8 - 11 wherein the xylitol is formulated for delivery by nebulization.

13. The use according to any preceding claim wherein the medicament is a solution comprising xylitol and a pharmaceutically acceptable carrier.

14. The use according to claim 13 wherein the pharmaceutically acceptable carrier is saline or water

15. The use according to any preceding claim wherein the medicament comprises a solution containing between approximately one and 64 parts xylitol, and 100 parts water.

16. The use according to any preceding claim wherein the non-human mammal is a head of cattle.
